# FASCICULE DE BREVET EUROPEEN

(11) **EP 0 579 528 B1**
(45) Date de publication et mention de la délivrance du brevet: **18.03.1998**
(21) Numéro de dépôt: 93401744.3
(22) Date de dépôt: 06.07.1993
(51) Int. Cl.: A61F 2/16

(54) **Implant intra-oculaire souple**
Biegsames Intraokularlinsenimplantat
Soft intra-ocular implant lens

(30) Priorité: 16.07.1992 FR 9208811
(43) Date de publication de la demande: 19.01.1994
(73) Titulaire: CORNEAL, F-75012 Paris (FR)
(72) Inventeur: Duchesne, Aldo, F-74410 St. Jorioz (FR); Bourgeois, Philippe, F-74000 Annecy (FR); Aron-Rosa, Danielle, 75016 Paris (FR)
(74) Mandataire: Dronne, Guy

(56) Documents cités:
- EP-A- 0 064 770
- EP-A- 0 255 759
- EP-A- 0 289 449
- EP-A- 0 308 130
- SU-A- 1 553 109
- US-A- 4 619 662

## Description

La présente invention concerne un implant intra-oculaire réalisé en un matériau souple.

Les implants intra-oculaires constituent des systèmes correcteurs de la vision humaine qui peuvent, dans un certain nombre de cas, se substituer à des lentilles cornéennes ou à des verres correcteurs externes. Dans d'autres cas, ces implants peuvent être combinés par exemple avec des verres correcteurs pour obtenir une correction convenable de la vision. Un implant intra-oculaire se compose essentiellement d'une partie optique de forme générale circulaire ou légèrement ovalisée qui forme le système optique correcteur proprement dit. Il comporte également une partie haptique qui sert à la mise en place et à la fixation de la partie optique à l'intérieur de l'oeil dans la position convenable.

L'opération dite de la cataracte se pratique maintenant le plus souvent selon la technique opératoire dite de phaco-émulsification. Cette technique permet l'ablation du cristallin opaque par l'introduction dans l'oeil d'une sonde à ultra-sons disposant d'un système d'irrigation/aspiration. Par l'action combinée des ultra-sons et du flux de BBS, on vient retirer le cristallin par émulsification.

Cette technique opératoire présente l'avantage par rapport aux techniques antérieures de ne nécessiter que la réalisation d'une incision de dimension réduite dans la cornée pour introduire dans l'oeil le matériel nécessaire à cette ablation. On comprend dès lors qu'il est intéressant de disposer d'implant intra-oculaire qui puisse être introduit à l'intérieur de l'oeil et mis en place par l'incision de dimension réduite qui est seule nécessaire lors de l'opération d'ablation du cristallin.

Avec les implants qui sont le plus souvent développés actuellement et qui sont réalisés en PMMA, c'est-à-dire en un matériau rigide, quelles que soient les précautions prises pour la réalisation des parties haptiques, l'incision doit nécessairement avoir une dimension suffisante pour laisser passer au moins la partie optique de l'implant dont le diamètre est de l'ordre de 6 mm.

Pour permettre de réduire encore les dimensions de l'implant dans la phase d'introduction à l'intérieur de l'oeil à travers l'incision, on a déjà proposé de réaliser des implants à l'aide de matériaux souples et hydrophiles connus sous le nom de HYDROGEL. Grâce à la très grande souplesse de ce matériau il est possible de plier ou de rouler l'implant, y compris sa partie optique, pour lui faire traverser l'incision, l'implant reprenant sa forme et ses dimensions normales lorsqu'il est mis en place dans l'oeil.

Cependant, en raison de sa très grande souplesse, la réalisation de la partie haptique de l'implant soulève des problèmes particuliers pour assurer une fixation et un positionnement convenable de l'implant à l'intérieur de l'oeil, notamment lorsque cet implant est mis en place dans le sac capsulaire. On comprend en particulier qu'il est nécessaire que la partie optique développe une force suffisante de maintien afin d'éviter les risques d'éjection de l'implant hors du sac capsulaire.

Un objet de la présente invention est de fournir un implant intra-oculaire monobloc, notamment pour sac capsulaire et réalisé en un matériau souple qui comporte une partie haptique assurant un positionnement et un maintien de l'implant amélioré par rapport au solution de l'art antérieur.

Un implant selon le préambule de la revendication 1 est connu de EP-A-255759.

L'implant selon l'invention est défini dans la revendication 1. Grâce à l'utilisation d'un matériau souple, la partie optique peut être pliée ou roulée pour faciliter l'insertion de l'implant dans l'oeil. Cependant, grâce notamment à la forme des portions de contact, l'implant est convenablement maintenu dans l'oeil, ce qui évite les risques d'éjection de l'implant lorsque celui-ci est mis en place dans le sac capsulaire.

Selon une autre caractéristique de l'invention, une des faces de la partie haptique est définie par une portion de surface torique dont l'axe est l'axe de révolution de la partie optique. Ainsi lorsque l'implant est mis en place dans le sac capsulaire et soumis à une pression, les éléments de fixation sont soumis à un effet d'enroulement qui est similaire à l'effet de voûte que l'on rencontre dans les implants de type classique à anse non fermée en forme de C ou de J.

Selon une autre caractéristique préférée de l'invention, chaque bras de raccordement a une épaisseur dans une direction perpendiculaire au plan de la partie optique qui va en augmentant depuis la périphérie de la partie optique vers la portion de contact et dont la largeur dans le plan de la portion optique va en diminuant de telle manière qu'on ait sensiblement une section constante desdits bras.

Selon ce mode préféré de réalisation, on a ainsi un moment d'inertie sensiblement constant sur toute la longueur du bras, ce qui assure une déformation convenable de celui-ci sous l'effet des contraintes.

D'autres caractéristiques et avantages de la présente invention apparaîtront mieux à la lecture de la description qui suit d'un mode de réalisation de l'invention donné à titre d'exemple non limitatif. La description se réfère aux figures annexées sur lesquelles:
- la figure 1 est une vue en plan de l'implant intra-oculaire;
- la figure 2 est une vue de côté de l'implant de la figure 1, et
- la figure 3 est une vue de côté de l'implant montrant plus précisément la forme de la partie haptique.

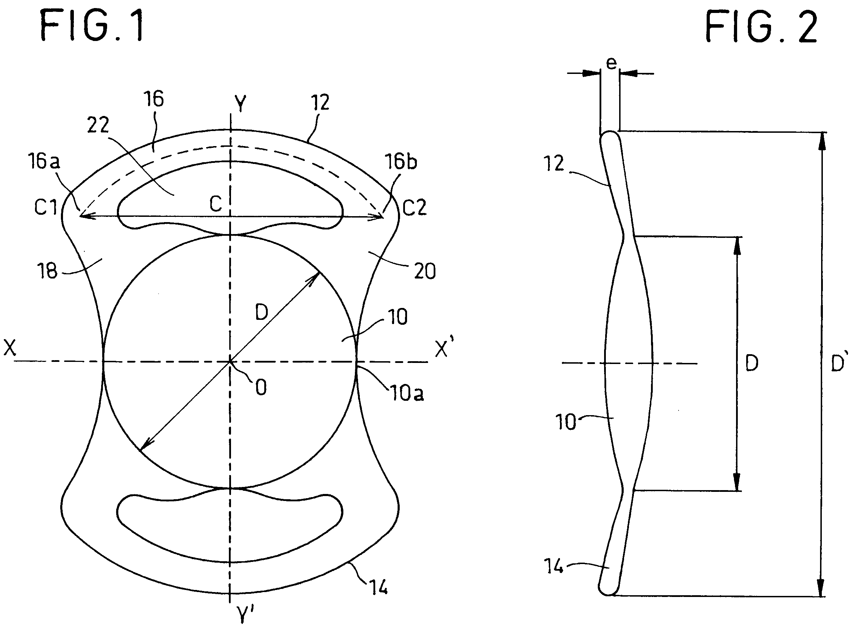

Comme le montre la figure 1, l'implant intra-oculaire comporte une partie optique 10 et deux éléments de fixation 12 et 14. La partie optique 10 est circulaire et de préférence son diamètre D est égal à 6 mm. La partie optique est constituée dans l'exemple représenté par deux surfaces convexes. A sa périphérie, la partie optique 10 a une épaisseur e qui est, dans l'exemple considéré, égale à 0,22 mm. L'ensemble de l'implant est réalisé en une seule pièce et de préférence en hydrogel.

Les deux éléments de fixation de la partie optique 12 et 14 sont identiques et sont symétriques par rapport à l'axe XX' passant par le centre O de la partie optique 10. On décrira donc uniquement l'élément de fixation 12 de la partie haptique. L'élément de fixation 12 est constitué par une portion de contact 16 et par deux bras de raccordement respectivement référencés 18 et 20 qui raccordent les extrémités 16a et 16b de la portion de contact à la périphérie de la partie haptique 10. On a représenté par C1, C2 la fibre neutre de la portion de contact. Celle-ci est bien sûr centrée au centre O de la partie optique et le diamètre externe correspondant D' est de préférence égal à 11 mm.

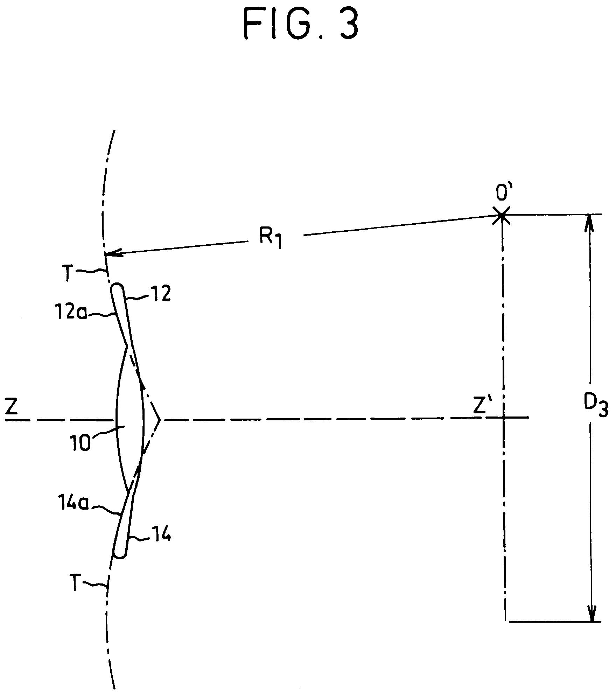

Comme le montre mieux la figure 3, une des faces 12a, 14a des éléments de fixation 12, 14 est disposée sur une portion de surface torique T représentée en pointillés. Cette portion de tore est définie par la rotation d'un cercle de centre O' et de rayon R1 autour de l'axe ZZ'. Lors de cette rotation, le centre O' décrit un cercle de diamètre D3. De préférence, le rayon R1 est compris entre 5 et 30mm et le diamètre D3 est compris entre 5 et 30 mm.

Selon une caractéristique de l'invention, la longueur de la corde C sous-tendant l'arc-de-cercle C1, C2 est au moins égale au diamètre D de la partie optique. Les bras de raccordement 18 et 20 partent tangentiellement de la périphérie 10a de la partie optique pour se raccorder également tangentiellement aux extrémités de la portion de contact 16 de façon à éviter tout point anguleux. Si l'on considère l'ensemble des deux bras 18 ou des deux bras 20, on voit que leur contour externe 24 ou 26 est concave et se raccorde tangentiellement à la périphérie 10a de la partie optique 10. On voit que les bras de raccordement 18 et 20 et de la portion de contact 16 délimitent une fenêtre 22 évidée. De préférence, l'épaisseur e de la portion de contact 16 dans une direction perpendiculaire au plan de la partie optique 10 est égale à 0,45 mm. On comprend qu'en conséquence les bras de raccordement ont une épaisseur qui va en augmentant depuis la périphérie 10a de la partie optique jusqu'aux extrémités 16a et 16b de la portion de contact 16. On voit également par la figure 1 que la largeur de chaque bras 18 ou 20 dans le plan de la partie optique 10 va en diminuant depuis la périphérie de la partie optique jusqu'aux extrémités de la portion de contact. On réalise ainsi des bras qui ont une section sensiblement constante, ce qui leur confère un moment d'inertie sensiblement constant sur toute leur longueur.

La présence des fenêtres évidées 22 procure au moins trois avantages. D'une part, elle facilite le pliage de l'implant autour du diamètre YY' de la partie optique constituant un axe de symétrie pour l'ensemble de l'implant. D'autre part, elle permet une déformation des portions de contact 16 qui peuvent ainsi s'adapter à des diamètres internes de l'oeil différents ou à la déformation du sac capsulaire du fait de l'ablation du cristallin. Enfin, ces fenêtres permettent la circulation de l'humeur aqueuse ou vitrée d'un côté à l'autre de l'implant.

Dans la description précédente, la partie haptique est constituée par deux éléments de fixation. On ne sortirait pas de l'invention si la partie haptique était constituée par trois ou quatre ensembles de fixation. Dans tous les cas, les ensembles de fixation sont angulairement répartis sur la périphérie de la partie optique.

Il faut ajouter que l'invention exploite seulement les propriétés de souplesse et de pliabilité de l'hydrogel et non ses propriétés hydrophiles. L'implant selon l'invention pourrait donc tout aussi bien être réalisé en un autre matériau souple tel que la silicone qui est également bien connue pour la réalisation d'implants intra-oculaires.

## Revendications

1. Implant intra-oculaire monobloc réalisé en un matériau souple et comportant une partie optique (10) sensiblement circulaire et une partie haptique, ladite partie haptique comportant deux éléments de fixation (12, 14) symétriques par rapport à un diamètre de la partie optique, chaque élément comportant une portion de contact (16) destinée à venir en contact avec la paroi interne de l'oeil et deux bras de raccordement (18, 20) pour raccorder respectivement chaque extrémité (16a, 16b) de la portion de contact à la périphérie (10a) de la partie optique, chaque portion de contact ayant sensiblement la forme d'un arc-de-cercle (C1, C2) caractérisé en ce que cet arc-de-cercle a sensiblement le même centre que la partie optique, la longueur de la corde soutendant ledit arc étant au moins égale au diamètre (D) de la partie optique.

2. Implant intra-oculaire selon la revendication 1, caractérisé en ce que une des faces (12a, 14a) de la partie haptique est définie par une portion de surface torique (T) ayant comme axe l'axe de révolution (ZZ') de la partie optique (10).

3. Implant intra-oculaire selon l'une quelconque des revendications 1 et 2, caractérisé en ce que chaque bras de raccordement (18, 20) a une épaisseur selon une direction perpendiculaire au plan de l'optique qui va en augmentant depuis la périphérie (10a) de la partie optique jusqu'à la portion de contact (16) et une largeur dans le plan de l'optique qui va en diminuant de la périphérie de la partie optique vers ladite portion de contact.

4. Implant intra-oculaire selon l'une quelconque des revendications 1 à 3, caractérisé en ce qu'il est réalisé en hydrogel.

5. Implant intra-oculaire selon l'une quelconque des revendications 1 à 3, caractérisé en ce qu'il est réalisé en silicone.

6. Implant intra-oculaire selon l'une quelconque des revendications 1 à 5, caractérisé en ce que le diamètre externe des deux portions de contact (16) est de l'ordre de 11 mm.

7. Implant intra-oculaire selon l'une quelconque des revendications 1 à 6, caractérisé en ce que le contour externe (24, 26) de deux bras de raccordement correspondants (18, 20) est concave et se raccorde tangentiellement au contour externe de la partie optique (10).

8. Implant intra-oculaire selon l'une quelconque des revendications 1 à 7, caractérisé en ce que l'épaisseur des portions de contact (16) est de 0,45 mm.

9. Implant intra-oculaire selon l'une quelconque des revendications 1 à 8, caractérisé en ce que la périphérie de ladite partie optique a une épaisseur de 0,22 mm.

## Claims

1. Intraocular implant in one piece made of a flexible material and including a substantially circular optic part (10) and a haptic part, the said haptic part including two fastening elements (12, 14) symmetrical with respect to a diameter of the optic part, each element including a contact portion (16) intended to come into contact with the inner wall of the eye, and two connecting arms (18, 20) for connecting each end (16a, 16b), respectively, of the contact portion to the periphery (10a) of the optic part, each contact portion having substantially the shape of an arc of a circle (C1, C2), characterized in that this arc of a circle has substantially the same centre as the optic part, the length of the chord subtending the said arc being at least equal to the diameter (D) of the optic part.

2. Intraocular implant according to Claim 1, characterized in that one of the faces (12a, 14a) of the haptic part in defined by a toric surface portion (T) having as its axis the axis of revolution (ZZ') of the optic part (10).

3. Intraocular implant according to either of Claims 1 and 2, characterized in that each connecting arm (18, 20) has a thickness, in a direction perpendicular to the plane of the optic, which increases from the periphery (10a) of the optic part as far as the contact portion (16), and a width, in the plane of the optic, which decreases from the periphery of the optic part towards the said contact portion.

4. Intraocular implant according to any one of Claims 1 to 3, characterized in that it is made of hydrogel.

5. Intraocular implant according to any one of Claims 1 to 3, characterized in that it is made of silicone.

6. Intraocular implant according to any one of Claims 1 to 5, characterized in that the external diameter of the two contact portions (16) is of the order of 11 mm.

7. Intraocular implant according to any one of Claims 1 to 6, characterized in that the external contour (24, 26) of two corresponding connecting arms (18, 20) is concave and connects tangentially to the external contour of the optic part (10).

8. Intraocular implant according to any one of Claims 1 to 7, characterized in that the thickness of the contact portions (16) is 0.45 mm.

9. Intraocular implant according to any one of Claim 1 to 8, characterized in that the periphery of the said optic part has a thickness of 0.22 mm.

## Patentansprüche

1. Intraokularimplantat in Einblockbauweise aus einem biegsamen Material, das ein im wesentlichen kreisförmiges optisches Teil (10) und ein haptisches Teil aufweist, wobei das haptische Teil zwei bezüglich eines Durchmessers des optischen Teils symmetrische Befestigungselemente (12, 14) aufweist, wobei jedes Element einen Kontaktabschnitt (16), der dazu bestimmt ist, mit der Innenwand des Auges in Kontakt zu kommen, und zwei Verbindungsarme (18, 20) aufweist, um jeweils jedes Ende (16a, 16b) des Kontaktabschnitts mit dem Rand (10a) des optischen Teils zu verbinden, wobei jeder Kontaktabschnitt im wesentlichen die Form eines Kreisbogens (C1, C2) hat, dadurch gekennzeichnet, daß dieser Kreisbogen im wesentlichen dasselbe Zentrum wie das optische Teil hat, wobei die Länge der den Bogen unterspannenden Kreissehne wenigstens gleich dem Durchmesser (D) des optischen Teils ist.

2. Intraokularimplantat nach Anspruch 1, dadurch gekennzeichnet, daß eine der Seiten (12a, 14a) des haptischen Teils durch einen Abschnitt mit torischer Fläche (T) bestimmt ist, der als Achse die Drehachse (ZZ') des optischen Teils (10) hat.

3. Intraokularimplantat nach einem der Ansprüche 1 und 2, dadurch gekennzeichnet, daß jeder Verbindungsarm (18, 20) eine Dicke in einer Richtung senkrecht zur Optikebene, die unter Anwachsen vom Rand (10a) des optischen Teils zum Kontaktabschnitt (16) verläuft und eine Breite in der Optikebene aufweist, die unter Verringerung vom Rand des optischen Teils zu dem Kontaktabschnitt verläuft.

4. Intraokularimplantat nach einem der Ansprüche 1 bis 3, dadurch gekennzeichnet, daß es aus Hydrogel hergestellt ist.

5. Intraokularimplantat nach einem der Ansprüche 1 bis 3, dadurch gekennzeichnet, daß es aus Silikon hergestellt ist.

6. Intraokularimplantat nach einem der Ansprüche 1 bis 5, dadurch gekennzeichnet, daß der äußere Durchmesser der beiden Kontaktabschnitte (16) im Bereich von 11 mm liegt.

7. Intraokularimplantat nach einem der Ansprüche 1 bis 6, dadurch gekennzeichnet, daß der äußere Umfang (24,26) der beiden entsprechenden Verbindungsarme (18,20) konkav ist und sich tangential mit dem äußeren Umfang des optischen Teils (10) verbindet.

8. Intraokularimplantat nach einem der Ansprüche 1 bis 7, dadurch gekennzeichnet, daß die Dicke der Kontaktabschnitte (16) 0,45 mm beträgt.

9. Intraokularimplantat nach einem der Ansprüche 1 bis 8, dadurch gekennzeichnet, daß der Rand des optischen Teils eine Dicke von 0,22 mm hat.
